# FASCICULE DE BREVET EUROPEEN

(11) **EP 0 980 378 B1**
(45) Date de publication et mention de la délivrance du brevet: **21.04.2004**
(21) Numéro de dépôt: 98924370.4
(22) Date de dépôt: 04.05.1998
(51) Int. Cl.: C07H 21/00, C07H 1/08

(54) **PROCEDE DE DETECTION DE LA PRESENCE DE MATIERES BIOLOGIQUES D'ORIGINE BOVINE, ET OLIGONUCLEOTIDES POUR SA MISE EN OEUVRE**
VERFAHREN ZUM NACHWEIS VON BIOLOGISCHEN MATERIALEN MIT BOVINER HERKUNFT, UND OLIGONUKLEOTIDEN DIE ES GEBRAUCHT
METHOD FOR DETECTING THE PRESENCE OF BIOLOGICAL MATTERS OF BOVINE ORIGIN, AND OLIGONUCLEOTIDES FOR ITS IMPLEMENTATION

(30) Priorité: 05.05.1997 FR 9705517
(43) Date de publication de la demande: 23.02.2000
(73) Titulaire: CENTRE NATIONAL DE LA RECHERCHE SCIENTIFIQUE, 75016 Paris Cédex (FR)
(72) Inventeur: HANNI, Catherine, F-69000 Lyon (FR); LAUDET, Vincent, F-69000 Lyon (FR); GRANGETTE, Corine, F-59152 Anstaing (FR); LANGE, Marc Résidence Le Corbusier, F-59777 Euralille (FR); PASTEAU, Stéphane, F-59130 Lamnersart (FR)
(74) Mandataire: Grosset-Fournier, Chantal Catherine
(86) Numéro de dépôt international: PCT/FR1998/000892
(87) Numéro de publication internationale: WO 1998/050401

(56) Documents cités:
- EP-A- 0 464 010
- EP-A- 0 546 762
- WO-A-94/14968
- WO-A-94/28177
- FR-A- 2 648 151
- US-A- 5 596 089
- SINCLAIR M C ET AL: "PCR strategies for isolation of the 5' end of an immunoglobulin -encoding bovine cDNA" GENE, vol. 1, no. 167, 1995, page 285-289 XP004043057

### LISTE DE SEQUENCES

(1) INFORMATION GENERALE:
   (i) DEPOSANT:
      (A) NOM: CENTRE NATIONAL DE LA RECHERCHE SCIENTIFIQUE
      (B) RUE: 3, rue Michel-Ange
      (C) VILLE: Paris
      (E) PAYS: FRANCE
      (F) CODE POSTAL: 75794 CEDEX 16
   (ii) TITRE DE L'INVENTION: PROCEDE DE DETECTION DE LA PRESENCE DE MATIERES BIOLOGIQUES D'ORIGINE BOVINE, ET OLIGONUCLEOTIDES POUR SA MISE EN OEUVRE
   (iii) NOMBRE DE SEQUENCES: 19
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 43 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 1:
   TAATGTCCAT GCTTATCATT ATGCTGGTGC TCAAGATGCA GTT 43
(2) INFORMATION POUR LA SEQ ID NO: 2:
   (i) CARACTERISTIQUES DE LA SEQUENCE:
      (A) LONGUEUR: 15 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 2: Y = T ou C
(2) INFORMATION POUR LA SEQ ID NO: 3:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 3: Y = T ou C
(2) INFORMATION POUR LA SEQ ID NO: 4:
   (i) CARACTERISTIQUES DE LA SEQUENCE:
      (A) LONGUEUR: 46 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 4:
   ATTATATGCC CCATGCATAT AAGCAAGTAC ATGACCTCTA TAGCAG 46
(2) INFORMATION POUR LA SEQ ID NO: 5:
   (i) CARACTERISTIQUES DE LA SEQUENCE:
      (A) LONGUEUR: 15 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 5:
   TAAGCAAGTA CATGA 15
(2) INFORMATION POUR LA SEQ ID NO: 6:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 6:
   GCATATAAGC AAGTACATGA 20
(2) INFORMATION POUR LA SEQ ID NO: 7:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 7:
   CTTGATAGTA TATCTATTAT ATATTCC 27
(2) INFORMATION POUR LA SEQ ID NO: 8:
   (i) CARACTERISTIQUES DE LA SEQUENCE:
      (A) LONGUEUR: 482 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 8: Y = C ou T
      R = A ou G
(2) INFORMATION POUR LA SEQ ID NO: 9:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 9:
   GAGCCTTATC AGTATTAAAT TTATC    25
(2) INFORMATION POUR LA SEQ ID NO: 10:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 10:
   CATTAATGTT ATGTACATTA 20
(2) INFORMATION POUR LA SEQ ID NO: 11:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 11:
   TTTCACGCGG CATGGTAATT 20
(2) INFORMATION POUR LA SEQ ID NO: 12:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 12:
   ATCCAATGAA TTTTACCAGG 20
(2) INFORMATION POUR LA SEQ ID NO: 13:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 13:
   GTCAATGGTC ACAGGACATA 20
(2) INFORMATION POUR LA SEQ ID NO: 14:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 14:
   ATTGACTTTG TTTGGAGTGC 20
(2) INFORMATION POUR LA SEQ ID NO: 15:
   (i) CARACTERISTIQUES DE LA SEQUENCE:
      (A) LONGUEUR: 159 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 15:
   GAGCCTTATC AGTATTAAAT TTATCAAAAA TCCCAATAAC TCAACACAGA ATTTGCACCC 60
   TAACCAAATA TTACAAACAC CACTAGCTAA CATAACACGC CCATACACAG ACCACAGAAT 120
   GAATTACCTA CGCAAGGGGT AATGTACATA ACATTAATG 159
(2) INFORMATION POUR LA SEQ ID NO: 16:
   (i) CARACTERISTIQUES DE LA SEQUENCE:
      (A) LONGUEUR: 153 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 16:
   GCATATAAGC AAGTACATGA CCTCTATAGC AGTACATAAT ACATATAATT ATTGACTGTA 60
   CATAGTACAT TATGTCAAAT TCATTCTTGA TAGTATATCT ATTATATATT CCTTACCATT 120
   AGATCACGAG CTTAATTACC ATGCCGCGTG AAA 153
(2) INFORMATION POUR LA SEQ ID NO: 17:
   (i) CARACTERISTIQUES DE LA SEQUENCE:
      (A) LONGUEUR: 265 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 17:
   ATCCAATGAA TTTTACCAGG CATCTGGTTC TTTCTTCAGG GCCATCTCAT CTAAAACGGT     60
   CCATTCTTTC CTCTTAAATA AGACATCTCG ATGGACTAAT GGCTAATCAG CCCATGCTCA    120
   CACATAACTG TGCTGTCATA CATTTGGTAT TTTTTTATTT TGGGGGATGC TTGGACTCAG    180
   CTATGGCCGT CAAAGGCCCT GACCCGGAGC ATCTATTGTA GCTGGACTTA ACTGCATCTT    240
   GAGCACCAGC ATAATGATAA GCATG                                          265
(2) INFORMATION POUR LA SEQ ID NO: 18:
   (i) CARACTERISTIQUES DE LA SEQUENCE:
      (A) LONGUEUR: 158 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 18:
   GTCAATGGTC ACAGGACATA AATTATATTA TATATCCCCC CTTCATAAAA ATTCCCCCCT     60
   TAAATATCTA CCACCACTTT TAACAGACTT TTCCCTAGAT ACTTATTTAA ATTTTTCACG    120
   CTTTCAATAC TCAATTTAGC ACTCCAAACA AAGTCAAT                            158
(2) INFORMATION POUR LA SEQ ID NO: 19:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 19:
   TAARCCGTGG GGGTCGCTAT CCAAT                                           25

## Description

La présente invention est relative à un procédé de détection de la présence de matières biologiques d'origine bovine dans un échantillon de matière organique.

Elle a en outre pour objet des oligonucléotides pour la mise en oeuvre de ce procédé.

En 1986, l'Encéphalopathie Spongiforme Bovine (ESB), ou maladie de "la vache folle", fut mise en évidence pour la première fois dans le cheptel bovin britannique. Depuis, plus de 100.000 cas cliniques ont été identifiés chez le bétail. Cette maladie, présente en Grande Bretagne, a une évolution endémique. Elle a été aussi observée dans différents pays européens : Irlande, Suisse, France, Danemark, Allemagne - où elle évolue de façon sporadique.

Le tableau clinique de la maladie est connu. L'agent infectieux responsable, appelé "prion" est caractérisé, entre autres propriétés, par une extrême résistance aux agents décontaminants classiques tels que la température, les radiations ou les détergents. Des études récentes ont par ailleurs montré la très grande résistance de l'agent infectieux dans les conditions "naturelles" et en particulier sa persistance dans les pâtures. Ainsi, l'infectiosité peut persister dans le sol pendant au moins trois ans.

Un des modes de transmission de l'agent infectieux est l'ingestion d'aliments contaminés, cette transmission pouvant par ailleurs se faire d'une espèce animale à l'autre.

L'analyse des données épidémiologiques a permis de découvrir l'origine de l'épidémie anglaise, due à une contamination par l'agent infectieux de farines de viandes et d'os (FVO) utilisées pour la fabrication d'additifs alimentaires destinés à l'alimentation des vaches laitières. Ces farines sont des sous-produits des ateliers d'équarrissage, issus du traitement de carcasses et des déchets provenant des abattoirs.

La structure du "prion" n'est pas connue à ce jour, et il n'existe aucun test permettant de le détecter.

Il est donc important de déterminer si une matière organique comprend des matières biologiques d'origine bovine, et est susceptible de contenir des "prions".

Dans le domaine agro-alimentaire, la caractérisation d'espèces animales a d'abord fait appel aux techniques biochimiques d'analyse des protéines (BARA et al. 1992, Trends in Food Science and Technology, 3, 69-72; SOTELO et al. 1993, Trends in Food Science and Technology, 4, 395-401; Hernandez et al., 1994, Food and Agricultural Immunology, 6, 95-104). Ces méthodes sont cependant peu spécifiques (électrophorèse) ou sont incompatibles avec la dénaturation des échantillons à analyser (immunoanalyse). Elles sont maintenant progressivement remplacées par les techniques d'analyse de l'ADN, molécule moins sensible que les protéines aux conditions physico-chimiques dénaturantes.

L'identification des principales espèces animales d'intérêt a tout d'abord été effectuée par la technique d'hybridation de sondes nucléiques (BUNTJER et al. 1995, Zeitschrift fuer Lebensmittel Untersuchung und Forschung 201 (6) : 577-582; MEYER et al., 1994, Fleischwirtschaft 74 (11) : 1237-1238; TSUMURA et al. 1992, Journal of Japanese Society of Food Science and Technology 39 (1) 60-63; EBBEHOJ et THOMSEN, 1991, Meat Science 30 (4): 359-366; BAUER et al., 1987, Archiv fuer Lebensmittelhygiene 38 (6): 172-174; EBBEHOJ et THOMSEN, 1991, Meat Science 30 (3): 221-234).

Cette technologie, d'utilisation délicate, est maintenant supplantée par la méthode de PCR (réaction de polymérisation en chaîne) qui a été utilisée pour caractériser des matières biologiques issues de diverses espèces animales.

Les seules méthodes d'amplification par PCR décrites chez le boeuf (Bos taurus) font appel à l'amplification de la région de l'ADN mitochondrial (ADNmt) codant pour un cytochrome au moyen d'amorces de PCR reconnaissant des séquences conservées chez les espèces de vertébrés, puis à leur caractérisation au moyen d'enzymes de restriction (RFLP) ou par séquençage (MEYER et al. 1995, Journal of AOAC - International 78 (6): 1542-1551; CHIKUNI et al. 1994, Animal Science and Technology, 65 (6): 571-579: GUGLICH at al. 1994, J. Forensic. Sci. 39 (2) : 353-361).

L'organisation et la séquence complète de l'ADN mitochondrial (ADNmt) bovin sont connues (ANDERSON et al. 1982, J. Mol. Biol. 156 (4): 683-717). A partir de ces données, plusieurs travaux ont été consacrés à l'étude de la variabilité génétique du génome mitochondrial de bovidés domestiques par analyse de polymorphisme de restriction (CHEN et al. 1995, Comp. Biochem. Physiol. B. Biochem. Mol. Biol. 111ᵢ (4) : 643-649; KIKKAWA et al. 1995, Biochem. Genet. 33 (1-2) : 51-60; BRADLEY et al. 1994, Anim. Genet. (4) : 265-271; AMANO et al. 1994, Anim. Genet. 25 (1) : 29-36; SUZUKI et al. 1993, Anim. Genet 24 (5) : 339-343: LAN et al. 1993, I. Chuan. Hsueh. Pao 20 (5) : 419-425; BHAT et al. 1990, Biochem. Genet 28 (7-8): 311-318 ; LOFTUS et al., 1994, Anim. Genet. 25:265-271) ou par séquençage (LOFTUS et al. 1994, Proc. Natl. Sci. USA 91 (7): 2757-2761; RON et al. 1993, Anim. Genet. 24 (3) : 183-186 ; BRADLEY et al., 1996, Proc. Natl. Acad. Sci. USA 93 : 5131-5135 ; BAILEY et al., 1996, Proc. R. Soc. Lond. B, 263:1467-1473).

Le séquençage partiel de la région de contrôle de l'ADNmt bovin a été effectué par ailleurs pour diverses races bovines européennes, africaines et indiennes, (LOFTUS et al., 1994 Proc. Natl. Sci. USA 91 (7): 2757-2761 ; BRADLEY et al., 1996, Proc. Natl. Acad. Sci. USA 93 : 5131-5135 ; BAILEY et al., 1996, Proc. R. Soc. Lond. B, 263:1467-1473).

Le brevet américain 5,596,089 concerne des séquences oligonucléotidiques bovines et porcines du gène nucléaire SRY, permettant la détermination moléculaire du sexe des bovins ou porcins. La méthode décrite dans ce document est une méthode de sexage des tissus bovins et porcins.

L'article de Sinclair et al. (*Gene,* 1995, vol. 1, n° 167, pages 285-289) concerne des stratégies utilisant la réaction de PCR afin d'isoler l'extrémité 5' de l'ADNc bovin codant pour l'immunoglobuline.

Il ressort donc de l'étude de l'état de la technique que des travaux d'analyses de l'ADN de certaines espèces et races bovines, ont déjà été effectués. Néanmoins, aucun des documents de l'état de la technique ne décrit de méthode spécifique et sensible d'amplification d'ADN bovin, permettant d'identifier des traces de matières biologiques d'origine bovine, applicable à toutes les races bovines et dans des matières organiques présentant des compositions très variées.

En effet, les techniques d'identification d'ADN bovin connues (par exemple analyse de génomes par RFLP ou PCR-RFLP réalisées sur des portions de séquences peu variables) présentent des inconvénients. Par ces méthodes peu spécifiques, il est souvent difficile d'analyser des mélanges d'ADN issus de différentes espèces, du fait du grand nombre de bandes, et des difficultés d'interprétation liées à cette caractéristique.

La faible sensibilité de certaines de ces techniques ne permet pas de mettre en évidence de façon fiable la présence de matières organiques d'origine bovine dans des substrats organiques très divers. Ces méthodes sont inapplicables lorsque l'ADN présent dans l'échantillon est dégradé sous forme de petits fragments de taille inférieure à environ 500 paires de bases.

Le problème de l'identification de matières organiques issues de toutes les races bovines est particulièrement crucial, car l'Encéphalite Spongiforme Bovine ne se limite pas aux races bovines européennes mais s'étend aux races africaines et indiennes (*Bos taurus* et *Bos indicus*).

Le demandeur s'est donc attaché à résoudre ces problèmes.

Il a montré que l'on pouvait détecter de manière spécifique et simple, et avec une grande sensibilité, la présence de matières biologiques d'origine bovine, quelle que soit la race bovine (*Bos taurus* et *Bos indicus*), dans des échantillons de matières organiques, en amplifiant de manière spécifique une séquence déterminée du génome bovin.

Dans sa forme la plus générale, la présente invention est donc relative à un procédé d'obtention d'un fragment d'ADN bovin présentant une taille et une séquence déterminées, spécifique des bovins, et en particulier des espèces Bos taurus et Bos indicus, à partir d'un échantillon de matière organique, procédé par lequel on amplifie, par réaction de polymérisation en chaîne, une séquence déterminée du génome bovin présente dans les génomes des bovins mais absente dans les génomes des autres espèces animales.

La présente invention a en outre pour objet un procédé de détection et d'identification de la présence de matières biologiques d'origine bovine dans un échantillon de matière organique caractérisé en ce que l'on détermine la présence d'ADN d'origine bovine dans ladite matière organique par amplification d'une séquence d'ADN spécifique du génome bovin.

On entend par matière organique toute matière solide ou liquide que l'on suppose avoir au moins partiellement une origine biologique.

Avantageusement, la séquence d'ADN est d'origine mitochondriale. Le choix d'une séquence mitochondriale est particulièrement avantageux, car, dans une cellule animale, il y a environ 100 à 1000 copies d'ADN mitochondrial pour une copie d'ADN nucléaire. En cas de dégradation de l'ADN, la probabilité de détecter de l'ADN mitochondrial est donc beaucoup plus importante que la probabilité de détecter de l'ADN nucléaire. En outre, l'ADN mitochondrial est plus résistant à la dégradation que l'ADN nucléaire. L'ADN mitochondrial peut donc être plus sûrement détecté dans des matières organiques dans lesquelles l'ADN est soumis à divers facteurs physiques (température, pression,...) chimiques (hydrolyse, oxydation,...) ou biochimiques tendant à sa dégradation.

Cette particularité se révèle particulièrement importante quand on cherche à détecter la présence de matières biologiques d'origine bovine dans des matières organiques qui ont subi de nombreuses transformations, par exemple en cosmétique, en agro-alimentaire, telles que les farines utilisées pour l'alimentation du bétail, les composts, les engrais et les fumiers etc...

L'invention est également avantageuse pour détecter la présence de matières biologiques d'origine bovine dans les substrats organiques suivants : les viandes crues, fumées, ou cuites, les granulés, le sang et les produits à base de sang, le lait et les produits à base de lait, les os et les produits à base d'os, les cuirs, les peaux, les ivoires, les poils, les cornes et les produits à base de corne, le guano, les fèces, les lisiers, les purins, la gélatine et les produits à base de gélatine, les produits cosmétiques et agro-alimentaires.

L'invention concerne des fragments d'ADN mitochondrial spécifiques du génome bovin, de tailles allant d'environ 500 paires de bases à environ 100 paires de bases notamment d'environ 152 à 480 paires de bases présentant une identité de séquence d'au moins 80% et de préférence d'au moins 90 % avec les régions homologues de la séquence de la région de contrôle de l'ADN mitochondrial déterminée par ANDERSON et al., 1982, J. Mol. Biol., 156, 683-717 et notamment allant de la position 15824 à la position 171, ces positions étant déterminées d'après la séquence complète de l'ADN mitochondrial du boeuf qui comprend 16338 nucléotides, déterminée par ANDERSON et al., 1982, J. Mol. Biol., 156, 683-717.

Préférentiellement, l'amplification de l'ADN est effectuée par la méthode d'amplification en chaîne par polymérase (PCR), comprenant une répétition d'un cycle constitué par les étapes suivantes :
- Chauffage de l'ADN extrait de l'échantillon de matière organique, de façon à séparer l'ADN en deux brins monocaténaires.
- Hybridation d'amorces oligonucléotidiques aux brins d'ADN monocaténaires à une température adéquate, et
- Elongation des amorces oligonucléotidiques par une polymérase à une température adéquate.

De manière particulièrement préférentielle, l'une des amorces est un oligonucléotide présentant une identité de séquence d'au moins 80%, préférentiellement d'au moins 90%, et avantageusement d'au moins 95%, avec un oligonucléotide constitué d'une séquence d'environ 15 à 25 nucléotides, notamment d'environ 20 à 25 nucléotides, comprise dans la séquence SEQ ID N°1 suivante (positions 136 à 178 d'après ANDERSON et al., 1982, J. Mol. Biol., 156, 683-717):

Cette première amorce peut être en particulier un oligonucléotide, ou un mélange d'oligonucléotides, comprenant la séquence SEQ ID N°2 suivante (positions 152 à 166 d'après ANDERSON et al., 1982, J. Mol. Biol., 156, 683-717) : dans laquelle Y est T ou C.

Elle est préférentiellement un oligonucléotide, ou un mélange d'oligonucléotides, présentant la séquence SEQ ID N°3 suivante (positions 152 à 171 d'après ANDERSON et al., 1982, J. Mol. Biol., 156, 683-717) : dans laquelle Y est T ou C.

La seconde amorce est un oligonucléotide présentant une identité de séquence d'au moins 80%, préférentiellement d'au moins 90%, et avantageusement d'au moins 95%, avec un oligonucléotide constitué d'une séquence d'environ 15 à 25 nucléotides, notamment d'environ 20 à 25 nucléotides comprise dans la séquence SEQ ID N°4 suivante (positions 16015 à 16060 d'après ANDERSON et al., 1982, J. Mol. Biol., 156, 683-717) :

Un tel oligonucléotide est préférentiellement celui comprenant la séquence SEQ ID N°5 suivante (positions 16034 à 16048 d'après ANDERSON et al., 1982, J. Mol. Biol., 156, 683-717) : ou encore préférentiellement celui présentant la séquence SEQ ID N°6 suivante (positions 16029 à 16048 d'après ANDERSON et al., 1982, J. Mol. Biol., 156, 683-717) :

Chacun des oligonucléotides SEQ ID N°1 à 3 est utilisé en couple avec l'un quelconque des oligonucléotides SEQ ID N°4 à 6. Le couple d'amorces le plus avantageux est le couple SEQ ID N°3 et SEQ ID N°6.

Selon un mode de mise en oeuvre particulièrement avantageux de la présente invention, au moins une partie des étapes d'hybridation des cycles constituant la réaction d'amplification est effectuée à une température d'environ 50°C à 58°C, notamment 50°C à 55°C. En outre, le demandeur a trouvé qu'une température d'environ 51 °C était particulièrement appropriée pour obtenir une amplification spécifique.

Un tel mode de mise en oeuvre permet d'obtenir une grande spécificité d'amplification.

Les températures des étapes de séparation des brins et d'élongation sont avantageusement respectivement d'environ 94°C et 72°C.

Le procédé décrit ci-dessus est spécifique car il ne donne aucune réaction d'amplification détectable en présence d'ADN d'origine autre que bovine (Bos taurus et Bos indicus). L'utilisation des amorces SEQ ID N°1 à SEQ ID N°6 ne donne naissance qu'à un fragment d'ADN d'environ 480 paires de bases. Ce fragment oligonucléotidique constitue un autre objet de la présente invention.

II présente avantageusement une identité de séquence d'au moins 80%, préférentiellement 90% et avantageusement 95% avec la séquence SEQ ID N°8 suivante (positions 16029 à 171 d'après la séquence de ANDERSON et al., 1982, J. Mol. Biol., 156, 683-717 qui comporte 16338 nucléotides) : R:AouG;Y:CouT;N:A,G,CouT

On notera à ce sujet que le demandeur a résolu un certain nombre de problèmes techniques pour la mise en oeuvre de ce procédé. Tout d'abord, le choix des amorces constituait un réel problème, car il fallait sélectionner des séquences d'une part communes aux différentes races bovines, et d'autre part s'hybridant de manière stable, dans des conditions physico-chimiques très variables représentatives de la grande diversité des matières organiques susceptibles de contenir des matières biologiques d' origine bovine.

Le demandeur a également mis au point d'autres amorces oligonucléotidiques présentées ci dessous et qui ont la caractéristique de pouvoir engendrer des fragments d'amplification plus petits, de taille inférieure à environ 200 paires de bases et supérieure à 100 paires de bases et avantageusement d'environ 150 paires de bases. Les séquences de ces amorces oligonucléotidiques sont les suivantes :

Les positions de ces amorces nucléotidiques étant définies d'après la séquence de ANDERSON et al., 1982, J. Mol. Biol., 156, 683-717, sont indiquées entre parenthèses.

L'invention concerne également des couples d'amorces oligonucléotidiques caractérisés en ce que les oligonucléotides les constituant sont choisis parmi ceux :
- présentant une identité de séquence d'au moins 80%, préférentiellement 90% et avantageusement 95% avec un oligonucléotide constitué d'une séquence d'environ 15 à 25 nucléotides, notamment 20 à 25 nucléotides, comprenant au moins 10 nucléotides contigus de la SEQ ID N°9 suivante :
- ou de la séquence SEQ ID N° 10 suivante :
- ou de la séquence SEQ ID N° 11 suivante :
- ou de la séquence SEQ ID N° 12 suivante :
- ou de la séquence SEQ ID N° 13 suivante :
- ou de la séquence SEQ ID N° 14 suivante :

Chacune des amorces oligonucléotides SEQ ID N°4 à 6 et SEQ ID N°9, 12 et 13 est utilisée en couple avec l'une quelconque des amorces oligonucléotides SEQ ID N°1 à 3 et SEQ ID N°10, 11 et 14. Les couples d'amorces oligonucléotides les plus avantageux sont les suivants : SEQ ID N°9 avec SEQ ID N°10, SEQ ID N°6 avec SEQ ID N°11, SEQ ID N°12 avec SEQ ID N°3, SEQ ID N°13 avec SEQ ID N°14.

Le couple d'amorces SEQ ID N°9 avec SEQ ID N°10 permet d'obtenir le fragment présentant avantageuement une identité de séquence d'au moins 80%, préférentiellement 90% et avantageusement 95% avec le fragment d'ADN suivant :

Le couple d'amorces SEQ ID N°6 avec SEQ ID N°11 permet d'obtenir le fragment présentant avantageuement une identité de séquence d'au moins 80%, préférentiellement 90% et avantageusement 95% avec le fragment d'ADN suivant :

Le couple d'amorces SEQ ID N°12 avec SEQ ID N°3 permet d'obtenir le fragment présentant avantageusement une identité de séquence d'au moins 80%, préférentiellement 90% et avantageusement 95% avec le fragment d'ADN suivant :

Le couple d'amorces SEQ ID N°13 avec SEQ ID N°14 permet d'obtenir le fragment présentant avantageusement une identité de séquence d'au moins 80%, préférentiellement 90% et avantageusement 95% avec le fragment d'ADN suivant :

Les produits d'amplification décrits ci dessus et notamment les séquences SEQ ID N°15 à SEQ ID N°18 peuvent être détectés lorsque une fraction importante de l'ADN est dégradé, à savoir après action des facteurs physiques, chimiques et/ou biochimiques décrits ci dessus et lors de transformations des substrats organiques.

L'expérimentateur recherche préférentiellement la présence SEQ ID N°8 décrite ci dessus à l'aide des amorces appropriées et dans le cas où la détection est négative, recherche les fragments de taille inférieure et notamment ceux d'environ 150 à 260 paires de bases engendrés notamment par l'utilisation des amorces SEQ ID N°9 à SEQ ID N°14.

L'utilisation des amorces SEQ ID N°9 à SEQ ID N°14 ne donne naissance qu'à des fragments d'ADN uniques d'environ 150 à 260 paires de bases. Le procédé décrit ci-dessus est spécifique car il ne donne aucune réaction d'amplification détectable en présence d'ADN d'origine autre que bovine. Ces fragments oligonucléotidiques constituent un autre objet de la présente invention.

L'unicité du produit d'amplification représente un autre avantage de la présente invention, celle ci permettant d'obtenir une sensibilité importante et facilitant grandement l'interprétation des résultats.

Le procédé selon la présente invention présente donc un grand nombre d'avantages par rapport aux techniques d'identification d'ADN bovin déjà connues.

Ainsi, le procédé décrit présente une grande simplicité d'interprétation, en raison de la production d'un seul et unique produit, spécifique de l'ADN bovin et qui donc ne se retrouve pas dans les produits d'amplification d'ADN d'autres espèces.

La lecture des profils de migration des produits d'amplification obtenus avec le procédé selon la présente invention consiste donc simplement à déterminer la présence d'une seule et unique bande de migration dans un gel d'électrophorèse. En l'absence d'une telle bande, on peut considérer qu'il n'y a pas de traces détectables d'ADN bovin. La présence d'une bande signifie au contraire que l'ADN bovin est présent dans l'échantillon, et donc que l'échantillon en question contient de la matière biologique d'origine bovine.

Le produit d'amplification peut être mis en évidence par toute méthode connue de l'homme du métier et, en particulier, par simple électrophorèse sur un gel d'agarose. Ce produit d'amplification peut être séquencé afin de déterminer la séquence nucléotidique et de confirmer son identité. Il peut également être mis en évidence par hybridation avec une sonde comprenant une partie oligonucléotidique et un marqueur. L'oligonucléotide constituant cette sonde comprend au minimum environ 15 nucléotides, préférentiellement au minimum environ 20 nucléotides.

Pour confirmer l'identité du fragment SEQ ID N°8 on utilise un oligonucléotide dont une partie de la séquence présente une identité d'au moins 80%, préférentiellement 90%, avec les séquences SEQ ID N°7 ou N°19 suivantes : où R est G ou A

L'identité des fragments SEQ ID N°15 à SEQ ID N°18 sera avantageusement confirmée par séquençage.

Le marqueur peut être tout marqueur connu de l'homme du métier, mais est préférentiellement la digoxygénine (DIG).

L'utilisation des sondes décrites ci-dessus pour mettre en évidence le produit de la réaction d'amplification SEQ ID N°8 est particulièrement avantageuse, car elle permet de confirmer l'origine bovine de ce produit d'amplification, et donc d'améliorer encore la spécificité et la sensibilité du procédé.

La présente invention est en outre relative à des oligonucléotides complémentaires et inverses/complémentaires des oligonucléotides décrits ci-dessus.

L'homme du métier pourra avantageusement se référer au manuel général de SAMBROOK et al. 1989, Molecular Cloning : A Laboratory Manual, Cold Spring Harbor Laboratory, Cold Spring Harbor NY., ou l'une de ses récentes rééditions, pour la mise en oeuvre des techniques de biologie moléculaire de la présente invention.

La présente invention permet de détecter la présence de composés d'origine bovine dans les produits utilisés en production agro-alimentaire et dans l'industrie des cosmétiques, tels que les viandes cuites ou crues, les granulés et farines utilisés pour l'alimentation du bétail, les composts, engrais et fumiers, les produits à base de sang, de poudre d'os, de cuir, de guano, les gélatines et les graisses animales.

La présente invention est illustrée, sans pour autant être limitée par les exemples qui suivent.

La figure 1 représente un gel d'agarose, coloré par du bromure d'éthidium. Les ADN de boeuf, de cheval, de mouton, de porc, de canard, de poulet et de dinde (respectivement puits 2 à 8) ont été amplifiés à l'aide des amorces SEQ ID N°6 (PBF9) et SEQ ID N°3 (PBR6). Le puits 1 correspond à un témoin négatif. Le produit d'amplification migre en formant une bande de 480 paires de bases.

La figure 2 est un autre gel d'agarose sur lequel ont été mis à migrer les produits d'amplification de l'ADN de différentes races européennes bovines : Bleu-blanc-belge (puits 2 et 3), Limousine (puits 4 et 5), Charolaise (puits 6 à 10), Normande (puits 11 et 12), Prim'Holstein (puits 13 à 16), Blonde d'Aquitaine (puits 17 à 20), Frisonne (puits 21), Croisée (puits 22 et 23) et Parthenaise (puits 24). Le puits 1 correspond à un témoin négatif.

La figure 3 représente le transfert par la technique Southern du gel d'agarose de la figure 1, hybridé à l'aide de la sonde SEQ ID N°7 (BH1) marquée.

La figure 4 est un gel d'agarose avec les produits d'amplification d'aliments pour bétails présentant des taux d'incorporation de farine bovine respectivement de 5; 2,5; 1; 0,5; 0,25; 0,1; 0,075; 0,06; 0,05; 0,01% (respectivement puits 2 à 11). Le puits 1 correspond à un témoin négatif.

La figure 5 est un transfert par la méthode Southern du gel représenté sur la figure 4, et hybridé par la sonde SEQ ID N°7 (BH1) marquée.

### EXEMPLE 1

### EXTRAIT ET DOSAGE DE L'ADN DE DIFFERENTES MATIERES ORGANIQUES.

Afin de pouvoir mettre au point une méthode de détection par amplification génique de composés d'origine bovine dans les produits utilisés en production agro-alimentaire et dans l'industrie des cosmétiques, les expériences décrites dans ce premier exemple ont été réalisées avec divers types d'échantillons représentant des sources potentielles de dissémination de l'ESB.

Les échantillons sélectionnés se répartissent en 12 groupes de produits finis, ou de produits entrant dans la composition des produits finis :

| | | |
|---|---|---|
| Amendements, composts | Engrais | Guano |
| Fumiers, fientes | Terreau | Granulés pour bétail |
| Farines pour bétail | Cuirs | Os et poudre d'os |
| Plumes | Sang | Gélatines, Graisse. |

Il est nécessaire pour pouvoir effectuer une analyse d'un échantillon quelconque par PCR de disposer de techniques d'extraction d'ADN. Etant donné la diversité des échantillons testés, plusieurs techniques sont mises en oeuvre :

### 1 ) Extraction d'ADN par la méthode au phénol/chloroforme.

Cette méthode s'adresse préférentiellement aux échantillons dont la teneur en composants d'origine animale est majoritaire (farines, granulés, os, sang, plumes, cuirs...).

Cette méthode fait appel à des techniques décrites par HÄNNI et al., 1990, C. R. Acad. Sci. Paris., 310, 365-370 et par HÄNNI et al., 1995, Nucl. Acids Res., 23, 881-882 décrivant l'extraction d'ADN à partir d'os et de dents.

On incube 0,5 g de poudre pendant trois heures à 56°C dans 2,5 ml de tampon de lyse de composition suivante :
Tris 100 mM, pH8
NaCl 100 mM
sarcosinate de sodium 1%.

Contenant 200 µg/ml de protéinase K, permettant de dégrader les protéines et de libérer les acides nucléiques.

Le lysat est ensuite extrait par un volume de phénol/chloroforme 1/1 pour éliminer la partie protéique du lysat. Une deuxième extraction est effectuée. La phase aqueuse est ensuite extraite par 1 volume de chloroforme.

L'ADN est alors précipité par 0,6 volume d'isopropanol et 0,1 volume d'acétate d'ammonium 2M à -20°C pendant un minimum de 2 heures. Il est ensuite récupéré par centrifugation (15 mn à 12000 rpm) puis lavé par de l'éthanol à 70°C (conservé à-20°C). Après évaporation de l'alcool résiduel (1 à 2 heures sous la hotte), l'ADN est dissous dans de l'eau pour préparation injectable (eau PPI ; le volume d'eau est fonction de la quantité d'ADN récupéré).

### 2 ) Extraction selon la méthode au CTAB (bromure de cétyltriméthylammonium).

Cette méthode décrite par MURRAY (Nucleic Acid Res. 8 (19), 4321-4325 (1980)) s'adresse préférentiellement aux échantillons contenant des débris d'origine végétale en mélange avec des produits d'origine animale (fumiers, composts, amendements, engrais, terreau).

1g d'échantillon est incubé 3 heures à 56°C dans 2,5 ml de tampon de lyse dont la composition est :
Tris 10 mM pH8
EDTA 0,1 mM
dodécylsulfate de sodium 1 %
protéinase K 100 µg/ml

450 µl de NaCl 5 M et 375 µl de CTAB 10% NaCl 0,7 M, préchauffés à 65°C, sont ajoutés et le tout incubé 20 mn à 65°C. Un volume de chloroforme est alors ajouté. Après agitation et centrifugation (10 mn à 12000 rpm à 4°C), la phase aqueuse est récupérée et réextraite une deuxième fois pour la rendre claire. Elle est de nouveau extraite par un volume de phénol/chloroforme. La suite de l'extraction s'effectue comme indiqué dans le paragraphe 1.

### 3 ) dosage quantitatif de l'ADN extrait.

La méthode de dosage utilisée est celle de LABARCA, (1980 Analytical Biochemistry 102, 344-352). Elle est basée sur la réaction d'intercalation d'une molécule, le Hoechst 33258 (2-(2-4 hydroxyphenyl)benzydalozyl)-6(1-méthyl-4-pipérazyl)-benzymidazol, 3HCl) dans la double hélice de l'ADN que l'on désire doser. Irradié à 356 nm, le complexe Hoechst 33258/ADN émet un signal de fluorescence caractéristique (458 nm).

L'émission de fluorescence est proportionnelle à la quantité d'ADN en solution ; la lecture s'effectue au moyen d'un fluorimètre (DyNA quant-Hoeffer). L'ADN de l'échantillon à doser est ajouté dans 2 ml de tampon Tris 10 mM, pH 7,4, EDTA 1 mM, NaCl 0,2 M contenant 1 µg de 33258/ml.

Par l'étalonnage de l'appareil à l'aide d'une solution d'ADN standard de concentration connue, la quantité d'ADN extrait de l'échantillon est mesurée.

Le tableau 1 ci-après montre que les techniques décrites permettent de mettre en évidence dans tous les échantillons de l'ADN en quantité suffisante pour être analysé par la méthode de PCR selon la présente invention.

### EXEMPLE 2 :

### AMPLIFICATION PAR PCR DE SEQUENCES D'ADNmt BOVIN A PARTIR DE L'ADN EXTRAIT DES ECHANTILLONS.

### 1 ) Purification de l'ADN extrait sur silice.

Pour pouvoir effectuer la réaction de PCR dans des conditions réactionnelles optimales, il est nécessaire de purifier au préalable l'ADN extrait. La purification décrite ci-dessous fait appel à une technique décrite par BOOM et al. (1990, J. Clin. Microbiol. 28, 495-503).

50 µl d'ADN brut, obtenu par l'une ou l'autre des méthodes détaillées dans l'exemple 1, sont dilués dans 400 µl d'un tampon obtenu par addition à 100 ml d'un tampon Tris 0,1 mM-pH 6,4 de 120g de thiocyanate de guanidinium, de 22 ml d'EDTA 0,2 M-pH8 et de 2,6 ml de Triton X100.

Cette solution est incubée 5 mn à température ambiante avec 300 µl de silice (Wizard DNA clean up System Promega ref A7280 : silice et minicolonnes). La silice est lavée sur colonne avec 2 ml d'isopropanol à 80%. La colonne est centrifugée 2 mn à 12000 rpm pour éliminer l'alcool résiduel, 50 µl d'eau PPI préchauffée à 70°C sont ajoutés pour éluer l'ADN fixé sur la silice (incubation de 5 à 30 mn à 70°C).

L'éluat contenant l'ADN récupéré par centrifugation (12000 rpm, 2 mn) est prêt à être utilisé pour la PCR.

Si nécessaire une deuxième purification sur silice est effectuée.

### 2 ) La réaction de PCR

La PCR a été mise en oeuvre avec les deux amorces oligonucléotidiques SEQ ID N°3 et SEQ ID N°6 présentant respectivement les séquences SEQ ID N°3 et SEQ ID N° 6.

Les amplifications ont été réalisées dans un volume total de 50 µl contenant :
5 µl d'un tampon Taqx10 (dépourvu de Mg+ +) commercialisé avec l'enzyme
5 µl d'un mélange dATP, dCTP, dGTP et dTTP (2 mM chacun)
3 µl de MgCl₂ 25 mM
0,5 µl d'albumine 20 mg/ml
5 µl de chacune des amorces SEQ ID N°3 et SEQ ID N°6 (10 µM chacun)
10 µl d'ADN de matrice de génome (de 100 ng à 1 µg)
0,2 µl de Taq ADN polymérase (5 unités/µl)
16,3 µl d'eau distillée stérile.

A l'exception de la matrice de génome, des amorces et de la Taq ADN polymérase, toutes les matières précédentes constituent la solution tampon de base préparée en volume et conservée à -20°C.

Toutes les amplifications ont été réalisées avec un appareil de commande de cycle thermique "Perkin-Elmer" avec le programme suivant de température :

| "Programme PCR" | |
|---|---|
| 1 cycle initial comprenant | 5 mn à 94°C |
| 9 cycles comprenant | 30 s à 94°C |
| | 30 s à 55°C (puis une température diminuée de |
| | 0,5°C chaque cycle jusqu'à 51°C) |
| | 30 s à 72°C |
| 32 cycles comprenant | 30 s à 94°C |
| | 30 s à 51°C |
| | 30 s à 72°C |
| 1 cycle final comprenant | 7 mn à 72°C |
| | de quelques minutes à plusieurs heures à 4°C. |

Les produits d'amplification sont analysés par électrophorèse sur gel d'agarose à 1,5% sous tension constante de 130 V pendant 45 mn, et à l'aide de bromure d'éthidium pour la visualisation des amplifiais obtenus.

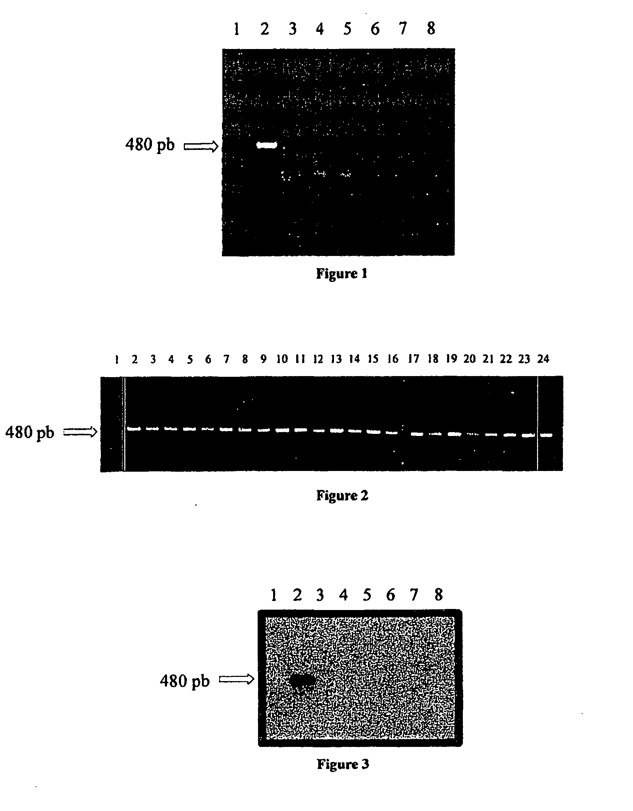

La réaction de PCR utilisant le couple d'amorces SEQ ID N°3 et SEQ ID N°6 a été effectuée sur une série d'extraits d'ADN obtenus à partir de divers tissus d'animaux et de végétaux. On observe un produit d'amplification unique (fragment d'une longueur de 480 paires de base - 480 pb-) pour les tissus bovins, et aucun produit pour tous les autres ADN testés (figure 1). Ainsi, les amorces SEQ ID N°3 et SEQ ID N°6 conviennent pour l'identification de l'ADN spécifique de bovin dans une application PCR.

En plus de ces résultats, la technique PCR utilisant ces deux amorces a été appliquée sur de l'ADN extrait de tissus d'animaux de races européennes bovines variées (Blanc-Bleu Belge, Limousine, Charolaise, Normande, Prim'Holstein, Charolaise croisée Normande, Blonde Aquitaine, Parthenaise). Tous les échantillons ont donné le même profil (un fragment unique de 480 pb) indiquant que la méthode peut être appliquée quelle que soit l'origine de l'ADN bovin analysé (figure 2).

### EXEMPLE 3 :

### CARACTERISATION DE L'ADN AMPLIFIE DANS LA REACTION DE PCR PAR LA TECHNIQUE DITE DE "SOUTHERN".

La confirmation du caractère "bovin" du produit d'amplification PCR (480 pb) est obtenue par l'hybridation à ce produit d'un sonde oligonucléotidique correspondant à la séquence d'identification BH1 (SEQ ID N°7). La sonde BH1 utilisée à cet effet est marquée à la digoxygénine (DIG) au moyen d'un kit commercial ("Dig oligonucléotide 3'-end labeling kit - Boehringer).

Après transfert par capillarité sur membrane de nylon, les produits d'amplification sont dénaturés avant fixation covalente à la membrane par UV. Après incubation de la membrane dans un tampon d'hybridation contenant la sonde BH1 marquée, il est procédé à des lavages, puis la sonde est révélée par un système anticorps anti-DIG couplés à la phosphatase alcaline en présence de chlorure de nitro blue tetrazolium et de 5-bromo, 4-chloro, 3-indolyl-phosphate. Les produits d'amplification sont ainsi caractérisés dans cette réaction par l'apparition d'une coloration brun-violet.

On constate, par l'utilisation de l'analyse par hybridation/transfert de type "Southern", que la sonde BH1 s'hybride spécifiquement à la séquence d'ADNmt bovin amplifiée au moyen des amorces SEQ ID N°3 et SEQ ID N°6, et pas à de l'ADN d'autres espèces animales et végétales (figure 3).

### EXEMPLE 4 :

### UTILISATION DES TECHNIQUES PCR ET "SOUTHERN" POUR LA DETECTION DE FARINES ANIMALES D'ORIGINE BOVINE DANS LES ALIMENTS DU BETAIL.

Les procédés des exemples 1, 2 et 3 ont été appliqués sur des échantillons d'aliments pour bétail (sous forme de farines ou de granulés), ne contenant pas de produits d'origine bovine, et auxquels a été incorporée expérimentalement de la farine de sous-produits bovins à des taux variant de 5 à 0,01 %.

Un signal spécifique, proportionnel au taux d'incorporation, a été obtenu par amplification PCR utilisant les amorces SEQ ID N°3 et SEQ ID N°6, sur tous les échantillons comprenant au moins 0,1 % de farine d'origine bovine.

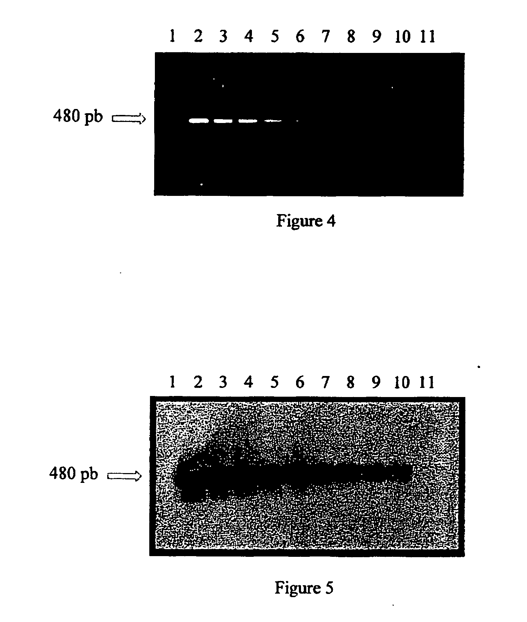

L'application de la technique "Southern" avec la sonde oligonucléotidique BH1 sur les produits d'amplification précédemment obtenus permet une détection spécifique des échantillons contenant de la farine des viande, avec cette fois-ci un taux limite de détection ramené à 0,05% (figure 4).

### EXEMPLE 5 :

### UTILISATION DES TECHNIQUES PCR ET "SOUTHERN" POUR LA DETECTION DE PRODUITS D'ORIGINE BOVINE DANS TOUS LES ECHANTILLONS.

Les procédés des exemples 1, 2 et 3 ont été appliqués sur des échantillons variés, susceptibles de contenir des taux variables des produits d'origine bovine, tels que les viandes cuites ou crues, les granulés et farines utilisées pour l'alimentation du bétail, les composts, engrais et fumiers, les produits à base de sang, de poudre d'os, de cuir, de guano, les gélatines et graisses animales.

Un signal spécifique a été obtenu avec tous les échantillons testés contenant des produits d'origine bovine (tableau 2 ci-après). On note une parfaite corrélation entre la présence reconnue de produit d'origine bovine dans les échantillons et le signal de PCR (présence de l'amplifiat de 480 pb s'hybridant avec la sonde BH1 marquée).

**Tableau 1 :**

| | | |
|---|---|---|
| Dosage au H33258 d'ADN extrait des échantillons | | |

| Résultats exprimés en µg d'ADN extrait à partir de 0,5 g de produit | | |
|---|---|---|
| Catégorie de Produit | Extraction Phénol/Chloroforme | Extraction CTAB |
| 1 Aliment* | 105.5 | 228.4 |
| 2 Aliment | 42.5 | 345.2 |
| 3 Aliment | 34.2 | 53.4 |
| 4 Compost | 3.6 | 287.4 |
| 5 Compost | 6.4 | 47.19 |
| 6 Cuir | 0.4 | 0.6 |
| 7 Cuir | 0.12 | 0.8 |
| 8 Engrais | 10.2 | 57.6 |
| 9 Engrais | 0.95 | 3.05 |
| 10 Engrais | 0.5 | 1.2 |
| 11 Farine | 291.4 | 346.2 |
| 12 Farine | 439.6 | 309.2 |
| 13 Farine | 322 | 278 |
| 14 Farine | 502.3 | 468.95 |
| 15 Farine | 198.6 | 154 |
| 16 Farine | 202 | 189.2 |
| 17 Fientes | 86.9 | 86.94 |
| 18 Fumier | 0.9 | 8.78 |
| 19 Fumier | 1.1 | 14.6 |
| 20 Fumier | 2.3 | 10.1 |
| 21 Fumier | 4.6 | 24.09 |
| 22 Graisse | 0.01 | 0.01 |
| 23 Gélatine | 0.01 | 0.01 |
| 24 Guano | 3.9 | 334.2 |
| 25 Os | 35.7 | 149.7 |
| 26 Os | 12.1 | 2.4 |
| 27 Os | 114.6 | 6.7 |
| 28 Plumes | 106.7 | 333.8 |
| 29 Plumes | 128.2 | 257.4 |
| 30 Sang | 1074.5 | 1147.8 |
| 31 Sang | 7.47 | 10.7 |
| 31 Terreau | 0.65 | 2.3 |
| 30 Terreau | 5.42 | 18.79 |

| | | |
|---|---|---|
| * Aliment granulé pour bétail | | |

**Tableau 2 :**

| Détection de produits d'origine bovin par la méthode de PCR utilisant les amorces PBF9 et PBR6 | | |
|---|---|---|
| Nature de l'échantillon analysé par PCR | Présence (P) ou absence (A) connue de sous-produits d'origine bovine dans l'échantillon analysé. | Réaction de PCR + : présence de l'amplifiat de 480 bp; - : absence de de l'amplifiat |
| 1 Aliment* | P | + |
| 2 Aliment | P | + |
| 3 Aliment | P | + |
| 4 Compost | A | - |
| 5 Compost | P | + |
| 6 Cuir | P | + |
| 7 Cuir | P | + |
| 8 Engrais | P | + |
| 9 Engrais | A | - |
| 10 Engrais | A | - |
| 11 Farine | A | - |
| 12 Farine | P | + |
| 13 Farine | P | + |
| 14 Farine | P | + |
| 15 Farine | P | + |
| 16 Farine | P | + |
| 17 Fientes | A | - |
| 18 Fumier | P | + |
| 19 Fumier | A | - |
| 20 Fumier | P | + |
| 21 Fumier | P | + |
| 22 Graisse | P | + |
| 23 Gélatine | P | + |
| 24 Guano | A | - |
| 25 Os | P | + |
| 26 Os | P | + |
| 27 Os | P | + |
| 28 Plumes | A | - |
| 29 Plumes | A | - |
| 30 Sang | P | + |
| 31 Sang | P | + |
| 31 Terreau | P | + |
| 30 Terreau | A | - |

| | | |
|---|---|---|
| *Aliment granulé pour bétail | | |

## Revendications

1. Oligonucléotides **caractérisés**
- **en ce qu'**ils présentent une identité de séquence d'au moins 80%, préférentiellement 90% et avantageusement 95% avec un oligonucléotide constitué d'une séquence d'environ 15 à 25 nucléotides, notamment 20 à 25 nucléotides, comprise dans la séquence SEQ ID N° 1 suivante :
- ou en ce qu'ils comprennent la séquence SEQ ID N° 2 suivante :
- ou en ce qu'ils sont constitués par la séquence SEQ ID N° 3 suivante :
dans laquelle Y est T ou C.

2. Oligonucléotides **caractérisés**
- **en ce qu'**ils présentent une identité de séquence d'au moins 80%, préférentiellement 90% et avantageusement 95% avec un oligonucléotide constitué d'une séquence d'environ 15 à 25 nucléotides, notamment 20 à 25 nucléotides, comprise dans la séquence SEQ ID N°4 suivante :
- ou en ce qu'ils comprennent la séquence SEQ ID N° 5 suivante :
- ou en ce qu'ils sont constitués par la séquence SEQ ID N° 6 suivante :

3. Couples d'amorces **caractérisés en ce qu'**ils sont constitués par l'un quelconque des oligonucléotides SEQ ID N° 1, SEQ ID N° 2, SEQ ID N° 3, selon la revendication 1, et l'un quelconque des oligonucléotides SEQ ID N° 4, SEQ ID N° 5, SEQ ID N° 6, selon la revendication 2, et avantageusement constitués par le couple d'oligonucléotides SEQ ID N° 3 et SEQ ID N° 6.

4. Couples d'amorces oligonucléotidiques **caractérisés en ce que** les oligonucléotides les constituant sont choisis parmi ceux :
- présentant une identité de séquence d'au moins 80%, préférentiellement 90% et avantageusement 95% avec un oligonucléotide constitué d'une séquence d'environ 15 à 25 nucléotides, notamment 20 à 25 nucléotides, comprenant au moins 10 nucléotides contigus de la séquence SEQ ID N°9 suivante :
- ou de la séquence SEQ ID N° 10 suivante :
- ou de la séquence SEQ ID N° 11 suivante :
- ou de la séquence SEQ ID N° 12 suivante :
- ou de la séquence SEQ ID N° 13 suivante :
- ou de la séquence SEQ ID N° 14 suivante :
et notamment les couples d'amorces suivants :
SEQ ID N°9 avec SEQ ID N°10, SEQ ID N°6 avec SEQ ID N°11,
SEQ ID N°12 avec SEQ ID N°3, SEQ ID N°13 avec SEQ ID N°14.

5. Oligonucléotide comprenant au minimum environ 15 nucléotides, préférentiellement au minimum environ 20 nucléotides, et dont au moins une partie de sa séquence présente une identité d'au moins 80%, préférentiellement 90% avec la séquence SEQ ID N°7 suivante : ou avec la séquence SEQ ID N° 19 suivante:

6. Sondes **caractérisées en ce qu'**elles comprennent des oligonucléotides selon la revendication 5 ou des oligonucléotides complémentaires ou inverse/complémentaires des oligonucléotides selon la revendication 5.

7. Procédé d'obtention d'un fragment d'ADN mitochondrial bovin présentant une taille et une séquence déterminées, spécifique des bovins, et en particulier des espèces Bos taurus et Bos indicus, à partir d'un échantillon de matière organique, procédé par lequel on amplifie, par réaction de polymérisation en chaîne, une séquence déterminée du génome bovin présente dans les génomes des bovins mais absente dans les génomes des autres espèces animales, ledit procédé étant **caractérisé en ce que** l'amplification est effectuée par la méthode d'amplification en chaîne par polymérase (PCR), comprenant une répétition du cycle des étapes suivantes :
- Chauffage de l'ADN extrait de l'échantillon de matière organique, de façon à séparer l'ADN en deux brins monocaténaires.
- Hybridation d'amorces oligonucléotidiques selon les revendications 1 à 4 aux brins d'ADN monocaténaires à une température adéquate, et
- Elongation des amorces oligonucléotidiques par une polymérase à une température adéquate.

8. Procédé de détection et d'identification de la présence de matières biologiques d'origine bovine dans un échantillon de matière organique **caractérisé en ce que** l'on détermine la présence d'ADN mitochondrial d'origine bovine dans ladite matière organique par amplification d'une séquence d'ADN mitochondrial spécifique du génome bovin, ledit procédé étant **caractérisé en ce que** l'amplification est effectuée par la méthode d'amplification en chaîne par polymérase (PCR), comprenant une répétition du cycle des étapes suivantes :
- Chauffage de l'ADN extrait de l'échantillon de matière organique, de façon à séparer l'ADN en deux brins monocaténaires.
- Hybridation d'amorces oligonucléotidiques selon les revendications 1 à 4 aux brins d'ADN monocaténaires à une température adéquate, et
- Elongation des amorces oligonucléotidiques par une polymérase à une température adéquate.

9. Procédé selon la revendication 7 ou 8, **caractérisé en ce que** la séquence d'ADN mitochondrial spécifique du génome bovin comprend environ 500 paires de bases à environ 100 paires de bases, notamment environ 152 à 480 paires de bases, et présente une identité de séquence d'au moins 80% et de préférence d'au moins 90 % avec les régions homologues de la séquence de la région de contrôle de l'ADN mitochondrial, et notamment allant de la position 15824 à la position 171, ces positions étant déterminées d'après la séquence complète de l'ADN mitochondrial du boeuf qui comprend 16338 nucléotides.

10. Procédé selon l'une des revendications 7 à 9, **caractérisé en ce que** la séquence amplifiée obtenue est détectée par hybridation avec une sonde ou par séquençage.

11. Procédé selon la revendication 10, **caractérisé en ce que** ladite sonde comprend au moins en partie l'oligonucléotide selon l'une des revendications 5 ou 6 et un marqueur.

12. Fragment d'ADN susceptible d'être obtenu par le procédé selon l'une des revendications 7 à 9, **caractérisé en ce qu'**il comprend environ 500 à environ 100 paires de bases.

13. Fragment selon la revendication 12, **caractérisé en ce qu'**il présente une identité de séquence d'au moins 80%, préférentiellement 90% et avantageusement 95% avec un oligonucléotide constitué d'une séquence d'environ 15 à 25 nucléotides, comprise dans la SEQ ID N°8 suivante : et notamment fragment de 480 paires de bases comprenant ou constitué par la SEQ ID N° 8 définie ci-dessus.

14. Fragments selon la revendication 12, **caractérisés en ce qu'**ils présentent une identité de séquence d'au moins 80%, préférentiellement 90% et avantageusement 95% avec un oligonucléotide constitué d'une séquence d'environ 15 à 25 nucléotides, comprise dans la SEQ ID N°15 suivante : ou dans la SEQ ID N°16 suivante : ou dans la SEQ ID N°17 suivante : ou dans la SEQ ID N°18 suivante : et notamment fragment de 159 paires de bases comprenant ou constitué par la SEQ ID N° 15 définie ci-dessus,
et notamment fragment de 153 paires de bases comprenant ou constitué par la SEQ ID N° 16 définie ci-dessus,
et notamment fragment de 265 paires de bases comprenant ou constitué par la SEQ ID N° 17 définie ci-dessus,
et notamment fragment de 158 paires de bases comprenant ou constitué par la SEQ ID N° 18 définie ci-dessus.

15. Utilisation du procédé selon l'une des revendications 7 à 11 pour la détection de matières biologiques d'origine bovine dans des produits tels que : les farines utilisées pour l'alimentation du bétail, les composts, les engrais et les fumiers, les viandes et mélanges de viandes crues, fumées, ou cuites, les granulés, le sang et les produits à base de sang, le lait et les produits à base de lait, les os et les produits à base d'os, les cuirs, les peaux, les ivoires, les poils, les cornes et les produits à base de corne, le guano, les fèces, les lisiers, les purins, la gélatine et les produits à base de gélatine, les produits cosmétiques, les produits utilisés en industrie agroalimentaire.

## Patentansprüche

1. Oligonucleotide, **dadurch gekennzeichnet, daß**
- sie mindestens eine 80proz. Sequenz-Identität, bevorzugt 90 Prozent und vorteilhaft 95 Prozent, mit einem Oligonucleotid haben, das aus einer Sequenz von ungefähr 15 bis 25 Nucleotide, vor allem 20 bis 25 Nucleotide besteht, welche Sequenz in der folgenden SEQ ID N° 1 eingeschlossen ist:
- oder daß sie die folgende SEQ ID N° 2 enthalten :
- oder daß sie aus der folgenden SEQ ID N° 3 bestehen :
worin Y T oder C bedeutet.

2. Oligonucleotide, **dadurch gekennzeichnet, daß**
- sie mindestens eine 80proz. Sequenz-Identität, bevorzugt 90 Prozent und vorteilhaft 95 Prozent, mit einem Oligonucleotid haben, das aus einer Sequenz von ungefähr 15 bis 25 Nucleotide, vor allem 20 bis 25 Nucleotide besteht, welche Sequenz in der folgenden SEQ ID N° 4 eingeschlossen ist:
- oder daß sie die folgende SEQ ID N° 5 enthalten :
- oder daß sie aus der folgenden SEQ ID N° 6 bestehen :

3. Primerpaar, **dadurch gekennzeichnet, daß** es aus irgendwelchen Oligonucleotiden der SEQ ID N° 1, SEQ ID N° 2, SEQ ID N° 3 nach Anspruch 1, und aus irgendwelchen Oligonucleotide der SEQ ID N° 4, SEQ ID N° 5, SEQ ID N° 6 nach Anspruch 2, sowie vorteilhaft aus zwei Nucleotiden der SEQ ID N° 3 und SEQ ID N° 6 bestehen.

4. Primerpaar, **dadurch gekennzeichnet, daß** die Oligonucleotide, aus denen es besteht, aus den folgenden gewählt werden :
- Oligonucleotide, die mindestens eine 80proz. Sequenz-Identität, bevorzugt 90 Prozent und vorteilhaft 95 Prozent, mit einem Oligonucleotid haben, das aus einer Sequenz von ungefähr 15 bis 25 Nucleotide, vor allem 20 bis 25 Nucleotide besteht, welche Sequenz mindestens 10 angrenzenden Nucleotide aus der folgenden SEQ ID N° 9 enthält:
- oder aus der folgenden SEQ ID N° 10:
- oder aus der folgenden SEQ ID N° 11:
- oder aus der folgenden SEQ ID N° 12:
- oder aus der folgenden SEQ ID N° 13:
- oder aus der folgenden SEQ ID N° 14:
und vor allem die folgenden Primerpaare:
SEQ ID N° 9 mit SEQ ID N° 10, SEQ ID N° 6 mit SEQ ID N° 11,
SEQ ID N° 12 mit SEQ ID N° 3, SEQ ID N° 13 mit SEQ ID N° 14.

5. Oligonucleotid, das mindestens 15 Nucleotide enthält, bevorzugt mindestens 20 Nucleotide, und worin mindestens ein Teil der Sequenz mindestens eine 80proz. Identität, bevorzugt 90 Prozent, mit der folgenden SEQ ID N° 7: oder mit der folgenden SEQ ID N° 19: hat.

6. Sonden, **dadurch gekennzeichnet, daß** sie Oligonucleotide nach Anspruch 5, oder komplementäre oder reverse/komplementäre Oligonucleotide zu den Oligonucleotiden nach Anspruch 5 enthalten.

7. Verfahren für die Herstellung eines Rinder-mitochondriales DNS-Fragment mit einer bestimmten Größe und Sequenz, das spezifisch für Rinder ist, insbesondere für Bos taurus und Bos indicus Spezies, aus einer organischen Substanzprobe, mit denem über eine Kettenpolymerisationsreaktion eine besondere Sequenz aus dem Rindergenom amplifiziert wird, welche Sequenz im Rindergenom anwesend ist, aber im Genom anderer Spezies nicht anwesend ist, welches Verfahren **dadurch gekennzeichnet, daß** die Amplification mit einer Polymerasekettenreaktion (PCR) durchgeführt wird, die die Wiederholung des Kreislaufs von den folgenden Schritten enthält:
- die Wärmung der aus der organischen Substanzprobe gewonnenen DNS um die DNS in zwei Einzelstränge zu trennen;
- die Hybridisierung der Oligonucleotid-Primer nach Ansprüche 1 bis 4 zu den DNS Einzelsträngen bei einer günstigen Temperatur, und
- die Verlängerung der Oligonucleotid-Primer mit einer Polymerase bei einer günstigen Temperatur.

8. Verfahren für die Detektion und Identifizierung der Anwesenheit von biologischen Substanzen aus Rinder in einer organischen Substanzprobe, **dadurch gekennzeichnet, daß** die Anwesenheit der Rinder-mitochondrialen DNS in der besagten organischen Substanz durch Amplifizierung einer rinderspezifischen mitochondrialen DNS-Sequenz detektiert wird, welches Verfahren **dadurch gekennzeichnet, daß** die Amplifizierung mit der Polymerasekettenreaktion durchgeführt wird, die die Wiederholung des Kreislaufs von den folgenden Schritten enthält:
- die Wärmung der aus der organischen Substanzprobe gewonnenen DNS um die DNS in zwei Einzelstränge zu trennen ;
- die Hybridisierung der Oligonucleotid-Primer nach Ansprüche 1 bis 4 zu den DNS Einzelsträngen bei einer günstigen Temperatur, und
- die Verlängerung der Oligonucleotid-Primer mit einer Polymerase bei einer günstigen Temperatur.

9. Verfahren nach Ansprüchen 7 oder 8, **dadurch gekennzeichnet, daß** die Rindergenom-spezifische mitochondriale DNS-Sequenz ungefähr 500 bis ungefähr 100 Basenpaare, vor allem ungefähr 152 bis 480 Basenpaare enthält, und mindestens eine 80proz. Sequenz-Identität, bevorzugt mindestens 90 Prozent, mit homologen Regionen in der Sequenz der mitochondrialen DNS-kontrollierenden Region, und vor allem von der Stelle 15834 bis der Stelle 171 hat, worin diese Stellen nach der gesamten Rinder-mitochondrialen DNS-Sequenz, die 16338 Nucleotide enthält, bestimmt werden.

10. Verfahren nach Ansprüchen 7 bis 9, **dadurch gekennzeichnet, daß** die erhaltene amplifizierte Sequenz durch Hybridisierung mit einer Probe oder durch Sequenzierung detektiert wird.

11. Verfahren nach Anspruch 10, **dadurch gekennzeichnet, daß** besagte Probe mindestens ein Teil des Oligonucleotides nach irgendwelchem Anspruch 5 oder 6 und ein Marker enthält.

12. DNS-Fragment, das durch das Verfahren nach irgendwelchem Anspruch 7 bis 9 erhalten werden kann, **dadurch gekennzeichnet, daß** es von ungefähr 500 bis ungefähr 100 Basenpaare enthält.

13. Fragment nach Anspruch 12, **dadurch gekennzeichnet, daß** es mindestens eine 80proz. Sequenz-Identität, bevorzugt 90 Prozent und vorteilhaft 95 Prozent, mit einem Oligonucleotid hat, das aus einer Sequenz besteht, die ungefähr 15 bis 25 Nucleotide enthält, die in der folgenden SEQ ID N° 8 eingeschlossen sind: und vor allem ein 480 Basenpaarefragment, das die wie oben definierte SEQ ID N° 8 enthält oder aus dieser Sequenz besteht.

14. Fragmente nach Anspruch 12, **dadurch gekennzeichnet, daß** sie mindestens eine 80proz. Sequenz-Identität, bevorzugt 90 Prozent und vorteilhaft 95 Prozent mit einem Nucleotid haben, das aus einer Sequenz besteht, die ungefähr 15 bis 25 Nucleotide enthält, die in der folgenden SEQ ID N° 15 eingeschlossen sind: oder in der folgenden SEQ ID N° 16: oder in der folgenden SEQ ID N° 17: oder in der folgenden SEQ ID N° 18: und vor allem ein 159 Basenpaarefragment, das die wie oben definierte SEQ ID N° 15 enthält oder aus dieser Sequenz besteht,
und vor allem ein 153 Basenpaarefragment, das die wie oben definierte SEQ ID N° 16 enthält oder aus dieser Sequenz besteht,
und vor allem ein 265 Basenpaarefragment, das die wie oben definierte SEQ ID N° 17 enthält oder aus dieser Sequenz besteht,
und vor allem ein 158 Basenpaarefragment, das die wie oben definierte SEQ ID N° 18 enthält oder aus dieser Sequenz besteht.

15. Anwendung des Verfahrens nach irgendwelchem Anspruch 7-11 für die Detektion von einer Rinder-biologischen Substanz in Produkte wie: Mehle, die als Rindfutter benutzt werden, Composts, Dünger und Düngemittel, rohe, geräucherte oder gekochte Fleische und Fleischmischungen, Granulate, Blut und blutgestützte Produkte, Milch und milchgestützte Produkte, Knochen und knochengestützte Produkte, Leder, Häute, Elfenbeine, Haare, Horn und homgestützte Produkte, Guano, Kot, Jaucheflüssigkeit, Jauche, Gelatine und gelatingestützte Produkte, Kosmetika und Produkte, die in der Ernährungsindustrie benutzt werden.

## Claims

1. Oligonucleotides, **characterised in that**
- they have at least 80% sequence identity, preferentially 90% and advantageously 95%, with an oligonucleotide which is made up of a sequence of about 15 to 25 nucleotides, preferably 20 to 25 nucleotides, contained in the following SEQ ID N° 1:
- or **in that** they comprise the following SEQ ID N° 2:
- or **in that** they are made up of the following SEQ ID N° 3:
wherein Y is T or C.

2. Oligonucleotides, **characterised in that**
- they have at least 80% sequence identity, preferentially 90% and advantageously 95%, with an oligonucleotide which is made up of a sequence of about 15 to 25 nucleotides, preferably 20 to 25 nucleotides, contained in the following SEQ ID N° 4:
- or **in that** they comprise the following SEQ ID N° 5:
- or **in that** they are made up of the following SEQ ID N° 6:

3. Pairs of primers, **characterised in that** they are made up of any of SEQ ID N° 1, SEQ ID N° 2, SEQ ID N° 3 oligonucleotides according to claim 1, and any of SEQ ID N° 4, SEQ ID N° 5, SEQ ID N° 6 oligonucleotides according to claim 2, and advantageously made up of the SEQ ID N° 3 and SEQ ID N° 6 oligonucleotide pair.

4. Pairs of oligonucleotide primers, **characterised in that** the oligonucleotides which make these up are chosen from those:
- having at least 80% sequence identity, preferentially 90% and advantageously 95%, with an oligonucleotide which is made up of a sequence of about 15 to 25 nucleotides, preferably 20 to 25 nucleotides, comprising at least 10 adjacent nucleotides from the following SEQ ID N° 9:
- or from the following SEQ ID N° 10:
- or from the following SEQ ID N° 11:
- or from the following SEQ ID N° 12:
- or from the following SEQ ID N° 13:
- or from the following SEQ ID N° 14:
and notably the following pairs of primers:
SEQ ID N° 9 with SEQ ID N° 10, SEQ ID N° 6 with SEQ ID N° 11,
SEQ ID N° 12 with SEQ ID N° 3, SEQ ID N° 13 with SEQ ID N° 14.

5. Oligonucleotide comprising at least about 15 nucleotides, preferentially at least about 20 nucleotides, and in which at least part of its sequence has at least 80% identity, preferentially 90%, with the following SEQ ID N° 7:, or with the following SEQ ID N° 19:

6. Probes as **characterised in that** they comprise oligonucleotides according to claim 5, or complementary or reverse/complementary nucleotides to the oligonucleotides according to claim 5.

7. A process for obtaining a bovine mitochondrial DNA fragment which has a defined size and sequence, and is specific to bovines, particularly the species Bos taurus and Bos indicus, from an organic matter sample, process by which a defined sequence of the bovine genome, present in bovine genome but absent from the genome of other animal species is amplified by a polymerase chain reaction, said process being **characterised in that** amplification is carried out with the polymerase chain reaction (PCR) method, comprising a repetition of the cycle of the following stages:
- heating of the DNA as extracted from the organic matter sample, such that the DNA is separated into two single strands;
- hybridizing of oligonucleotide primers according to claims 1 to 4 with the single DNA strands at an appropriate temperature, and
- elongation of the oligonucleotide primers by a polymerase at an appropriate temperature.

8. Process for the detection and identification of the presence of bovine biological matter in an organic matter sample, **characterised in that** the presence of bovine mitochondrial DNA is detected in said organic matter by amplification of a mitochondrial DNA sequence which is specific to the bovine genome, said process being **characterised in that** amplification is carried out using the polymerase chain reaction (PCR) method, comprising a repetition of the cycle of the following stages:
- heating of the DNA as extracted from the organic matter sample, such that the DNA is separated into two single strands;
- hybridizing of oligonucleotide primers according to claims 1 to 4 with the single DNA strands at an appropriate temperature, and
- elongation of the oligonucleotide primers by a polymerase at an appropriate temperature.

9. Process according to claim 7 or 8, **characterised in that** the mitochondrial DNA sequence which is specific to the bovine genome includes from about 500 base pairs to about 100 base pairs, preferably from about 152 to 480 base pairs, and has at least 80% sequence identity, and preferably at least 90%, with homologous regions in the sequence of the mitochondrial DNA control region, and notably from position 15834 to position 171, these positions being determined according to the complete bovine mitochondrial DNA sequence, which comprises 16338 nucleotides.

10. Process according to claims 7 to 9, **characterised in that** the obtained amplified sequence is detected by hybridisation with a probe or by sequencing.

11. Process according to claim 10, **characterised in that** said probe includes at least part of the oligonucleotide according to any of claims 5 or 6 and a marker.

12. DNA fragment which may be obtained by the process according to any of claims 7-9, **characterised in that** it includes from about 500 to about 100 base pairs.

13. Fragment according to claim 12, **characterised in that** it has at least 80% sequence identity, preferentially 90%, and advantageously 95%, with an oligonucleotide which is made up of a sequence which includes from about 15 to 25 nucleotides, as comprised in the following SEQ ID N° 8: and preferably a 480 base pair fragment comprising or made up of SEQ ID N° 8 as defined hereabove.

14. Fragments according to claim 12, **characterised in that** they have at least 80% sequence identity, preferentially 90%, and advantageously 95%, with an oligonucleotide which is made up of a sequence of about 15 to 25 nucleotides, as comprised in the following SEQ ID N° 15: or in the following SEQ ID N° 16 : or in following SEQ ID N° 17 : or in the following SEQ ID N° 18 : and preferably a 159 base pair fragment comprising or made up of SEQ ID N° 15 as defined hereabove,
and preferably a 153 base pair fragment comprising or made up of SEQ ID N° 16 as defined hereabove,
and preferably a 265 base pair fragment comprising or made up of SEQ ID N° 17 as defined herabove,
and preferably a 158 base pair fragment comprising or made up of SEQ ID N° 18 as defined hereabove.

15. Use of a process according to any of claims 7 to 11, for the detection of bovine biological matter in products such as: meals used for feeding cattle, composts, manures and dungs, raw, smoked or cooked meats and meat mixtures, pellets, blood and blood-based products, milk and milk-based products, bone and bone-based products, hides, skins, ivories, furs, horns and horn-based products, guano, faeces, semi-liquid manures, liquid manures, gelatine and gelatine-based products, cosmetics, and products used in the food industry.
